# EUROPÄISCHE PATENTSCHRIFT

(11) **EP 1 230 371 B1**
(45) Veröffentlichungstag und Bekanntmachung des Hinweises auf die Patenterteilung: **31.05.2006**
(21) Anmeldenummer: 00974439.2
(22) Anmeldetag: 23.10.2000
(51) Int. Cl.: C12N 15/24, C07K 14/54, C12N 15/62, C12N 1/19

(54) **VERFAHREN ZUR HERSTELLUNG VON REKOMBINANTEM INTERLEUKIN-8 UND INTERLEUKIN-8 MUTEINEN UND DEREN VERWENDUNG ZUM AUFFINDEN VON ANTAGONISTEN FÜR IL-8 REZEPTOREN**
METHOD FOR PRODUCING RECOMBINANT INTERLEUCIN-8 AND INTERLEUCIN-8 MUTEINS AND THEIR USE FOR SCREENING IL-8 RECEPTOR ANTAGONISTS
PROCEDE DE PRODUCTION D'INTERLEUKINE-8 ET DE MUTEINES D'INTERLEUKINE-8 DE RECOMBINAISON ET LEUR UTILISATION POUR DECOUVRIR DES ANTAGONISTES DES RECEPTEURS D'IL-8

(30) Priorität: 02.11.1999 DE 19952622
(43) Veröffentlichungstag der Anmeldung: 14.08.2002
(73) Patentinhaber: Bayer HealthCare AG, 51368 Leverkusen (DE)
(72) Erfinder: APELER, Heiner, 42111 Wuppertal (DE); SCNEIDER, Karl-Heinz, 42115 Wuppertal (DE); GOTTSCHALK, Uwe, 42555 Velbert (DE); SCHRÖDER, Werner, 42113 Wuppertal (DE)
(86) Internationale Anmeldenummer: PCT/EP2000/010402
(87) Internationale Veröffentlichungsnummer: WO 2001/032879

(56) Entgegenhaltungen:
- WO-A-89/04325
- US-A- 5 407 822
- US-A- 5 707 831
- US-A- 5 871 723
- SCHRAUFSTATTER INGRID U ET AL: "The role of Tyr-13 and Lys-15 of interleukin-8 in the high affinity interaction with the interleukin-8 receptor type A." JOURNAL OF BIOLOGICAL CHEMISTRY, Bd. 270, Nr. 18, 1995, Seiten 10428-10431, XP002166109 ISSN: 0021-9258
- HAMMOND W M E ET AL: "RECEPTOR RECOGNITION AND SPECIFICITY OF INTERLEUKIN-8 IS DETERMINED BY RESIDUES THAT CLUSTER NEAR A SURFACE-ACCESSIBLE HYDROPHOBIC POCKET" JOURNAL OF BIOLOGICAL CHEMISTRY,US,AMERICAN SOCIETY OF BIOLOGICAL CHEMISTS, BALTIMORE, MD, Bd. 271, Nr. 14, 5. April 1996 (1996-04-05), Seiten 8228-8235, XP002166116 ISSN: 0021-9258

### SEQUENZPROTOKOLL

<110> Bayer AG
<120> Verfahren zur Herstellung von rekombinantem Interleukin-8 und Interleukin-8 Muteinen
<130> Verf. zur Herstellung IL-8
<140>
   <141>
<160> 11
<170> Patent In Vers. 2.0
<210> 1
   <211> 72
   <212> PRT
   <213> Human
<400> 1
   Ser Ala Lys Thr Val Arg Cys Gln Cys Ile Lys Thr Tyr Ser Lys Pro
    1               5                  10                  15
   Phe His Pro Lys Phe Ile Lys Glu Leu Arg Val Ile Glu Ser Gly Pro
               20                  25                  30
   His Cys Ala Asn Thr Glu Ile Ile Val Lys Leu Ser Asp Gly Arg Glu
           35                  40                  45
   Leu Cys Leu Asp Pro Lys Glu Asn Trp Val Gln Arg Val Val Glu Lys
       50                  55                  60
   Phe Leu Lys Arg Ala Glu Asn Ser
   65                  70
<210> 2
   <211> 244
   <212> DNA
   <213> Human
<400> 2
   aagcttggat aaaagaagtg ctaaagaact tagatgtcaa tgcataaaga catactccaa 60
   acctttccac cccaaattta tcaaagagct cagagtgatt gagagtggac cacactgcgc 120
   caacacagaa attattgtaa aactttctga tggaagagag ctgtgtctgg accccaagga 180
   aaactgggtg cagagggttg tggagaagtt tttgaagagg gctgagaact catgataagg 240
   atcc                                                              244
<210> 3
   <211> 51
   <212> DNA
   <213> Human
<400> 3
   ggaagcttgg ataaaagaag tgctaaaact gtcagatgtc aatgcataaa g 51
<210> 4
   <211> 20
   <212> DNA
   <213> Human
<400> 4
   cttctgttcg gagattaccg 20
<210> 5
   <211> 244
   <212> DNA
   <213> Human
<400> 5
   aagcttggat aaaagaagtg ctaaaactgt cagatgtcaa tgcataaaga catactccaa 60
   acctttccac cccaaattta tcaaagagct cagagtgatt gagagtggac cacactgcgc 120
   caacacagaa attattgtaa aactttctga tggaagagag ctgtgtctgg accccaagga 180
   aaactgggtg cagagggttg tggagaagtt tttgaagagg gctgagaact catgataagg 240
   atcc                                                              244
<210> 6
   <211> 72
   <212> PRT
   <213> Human
<400> 6
   Ser Ala Lys Asp Leu Gln Cys Gln Cys Ile Lys Thr Tyr Ser Lys Pro
   1               5                   10                  15
   Phe His Pro Lys Phe Ile Lys Glu Leu Arg Val Ile Glu Ser Gly Pro
               20                  25                  30
   His Cys Ala Asn Thr Glu Ile Ile Val Lys Leu Ser Asp Gly Arg Glu
           35                  40                  45
   Leu Cys Leu Asp Pro Lys Glu Asn Trp Val Gln Arg Val Val Glu Lys
       50                  55                  60
   Phe Leu Lys Arg Ala Glu Asn Ser
   65                  70
<210> 7
   <211> 51
   <212> DNA
   <213> Human
<400> 7
   ggaagcttgg ataaaagaag tgctaaagat ttgcaatgtc aatgcataaa g 51
<210> 8
   <211> 244
   <212> DNA
   <213> Human
<400> 8
   aagcttggat aaaagaagtg ctaaagattt gcaatgtcaa tgcataaaga catactccaa 60
   acctttccac cccaaattta tcaaagagct cagagtgatt gagagtggac cacactgcgc 120
   caacacagaa attattgtaa aactttctga tggaagagag ctgtgtctgg accccaagga 180
   aaactgggtg cagagggttg tggagaagtt tttgaagagg gctgagaact catgataagg 240
   atcc 244
<210> 9
   <211> 72
   <212> PRT
   <213> Human
<400> 9
   Ser Ala Lys Glu Leu Arg Cys Gln Cys Ile Lys Thr His Ser Lys Pro
   1 5 10 15
   Phe His Pro Lys Phe Ile Lys Glu Leu Arg Val Ile Glu Ser Gly Pro
   20 25 30
   His Cys Ala Asn Thr Glu Ile Ile Val Lys Leu Ser Asp Gly Arg Glu
   35 40 45
   Leu Cys Leu Asp Pro Lys Glu Asn Trp Val Gln Arg Val Val Glu Lys
   50 55 60
   Phe Leu Lys Arg Ala Glu Asn Ser
   65 70
<210> 10
   <211> 72
   <212> DNA
   <213> Human
<400> 10
   ggaagcttgg ataaaagaag tgctaaagaa cttagatgtc aatgcataaa gacacattcc 60
   aaacctttcc ac 72
<210> 11
   <211> 244
   <212> DNA
   <213> Human
<400> 11
   aagcttggat aaaagaagtg ctaaagaact tagatgtcaa tgcataaaga cacattccaa 60
   acctttccac cccaaattta tcaaagagct cagagtgatt gagagtggac cacactgcgc 120
   caacacagaa attattgtaa aactttctga tggaagagag ctgtgtctgg accccaagga 180
   aaactgggtg cagagggttg tggagaagtt tttgaagagg gctgagaact catgataagg 240
   atcc 244

## Beschreibung

Die Erfindung betrifft ein Verfahren zur Herstellung von rekombinantem Interleukin-8 (IL-8) und Interleukin-8 Muteinen und deren Verwendung zum Auffinden von IL-8 Antagonisten für die IL-8 Rezeptoren CXCR1 und CXCR2. In besonderer Weise wird ein Verfahren zur Herstellung von IL-8 Y13H, IL-8 TVR und IL-8 DLQ beschrieben.

Die Familie der Chemokine umfasst chemotaktische Cytokine für Leukozyten mit einem Molekulargewicht von 8 bis 10 kD. Die dreidimensionale Struktur mehrerer Chemokine ist bekannt. Chemokine enthalten zwei Disulfidbrücken. Auf Grund eines konservierten C-X-C-bzw. C-C-Motivs am N-Termirius unterscheidet man die Unterfamilien der CXC- bzw. CC-Chemokine (Baggiolini et al., Advances in Immunology 55, 97 - 179, 1993). Zu den CXC-Chemokinen zählen u.a. Interleukin-8, γ-Interferon-induzierbares-Protein-10 (IP-10), Plättchen Faktor 4 (PF4), GRO (growth related oncogene) α, β, γ, Monokin induziert durch gamma-Interferon (MIG) und das Epitheliale Neutrophil aktivierende Protein-78 (ENA-78).

Neben der akuten inflammatorischen Wirkung auf Neutrophile Granulozyten sind für einzelne C XC-Chemokine auch a ngiogene und a ngiostatische Effekte beschrieben. Humanes re-kombinantes IP-10 und PF4 zählen zu den CXC-Chemokinen mit angiostatischer Wirkung. Diesen Chemokinen fehlt eine charakteristische Sequenz Glu-Leu-Arg (ELR Motiv) am N-terminalen Ende des Proteins. Interleukin-8 zählt zu den CXC-Chemokinen mit ELR-Motiv, das angiogene Wirkungen induziert. Humane Interleukin-8 Muteine, bei denen das ELR (Glu-Leu-Arg) Motiv durch die Aminosäuren Thr-Val-Arg (TVR) oder Asp-Leu-Asn (DLQ) ersetzt ist, zeichnen sich durch eine angiostatische Wirkung aus (Strieter et al., J. Biol. Chem. 270, 27348 - 27357, 1995 und Strieter et al., United States Patent 5,871,723).

SCHRAUFSTATTER INGRID U ET AL beschreibt in: 'The role of Tyr-13 and Lys-15 of interleukin-8 in the high affinity interaction with the interleukin-8 receptor type A'.

(JOURNAL OF BIOLOGICAL CHEMISTRY, Bd. 270, Nr. 18, 1995, Seiten 10428-10431, XP002166109 ISSN: 00221-9258)

Interleukin-8 Muteine und deren Interaktion mit dem IL8-Rezeptor. Dieses Zitat enthält keinen Hinweis auf die Verwendung von im Vergleich zu Wildtyp IL-8 schwächer affinen IL-8 Muteinen in Screening Ansätzen zum Auffinden nieder- und hochmolekularer IL-8 Antagonisten für die IL-8 Rezeptoren CXCR1 und CXCR2.

Die rekombinante Herstellung der Interleukin-8 Muteine erfolgte über die Expression ein Fusion mit dem Maltose Bindeprotein (MBP), der Reinigung über eine Amylose Matrix (New England Biolabs), der proteolytischen Spaltung des Fusionsproteins und anschließenden Feinreinigung des gewünschten Muteins (Strieter et al., J. Biol. Chem. 270, 27348 - 27357, 1995). Eine Produktion von Interleukin-8 und Interleukin-8 Muteinen im technischen Maßstab ist mit diesem Verfahren nicht möglich.

Überraschenderweise wurde gefunden, dass eine Expression von Interleukin-8 und Interleukin-8 Muteinen mit korrekter N-terminaler Sequenz in Hefe-Sekretionsvektoren mit MFα1 Leader Sequenz möglich ist. Das so hergestellte IL-8 und die so hergestellten Muteine erhält man in höherer Reinheit und frei von Endotoxinen. Die hergestellten Interleukin-8 Muteine TVR und DLQ eignen sich zur Behandlung von Krebserkrankungen. Das Interleukin-8 Mutein Y13H ist außerdem geeignet zur Identifizierung von niedermolekularen Interleukin-8 Antagonisten am CXCR1 und CXCR2 Rezeptor.

Nachstehend werden die im Verlauf der Klonierung, Fermentation, Reinigung und biochemischen Charakterisierung der rekombinanten Interleukin-8 Muteine eingesetzten Methoden aufgeführt.

### Methoden

### Enzyme

Die verwendeten Enzyme (Restriktionsendonucleasen, Alkalische Phosphatase aus Kälberdarm und T4 DNA Ligase) wurden von den Firmen Boehringer Mannheim und GIBCO/BRL bezogen und nach Vorschriften der Hersteller eingesetzt.

### Molekularbiologische Techniken

Routinemäßige Klonierungsarbeiten, wie z.B. die Isolation von Plasmid-DNA aus E. coli (sog. miniprep) und die Transformation von E. coli mit Plasmid-DNA wurden nach Sambrook et al. (Molecular Cloning, Cold Spring Harbor, 1989) durchgeführt. Als Wirtsorganismus für Transformationen wurde der E. coli Stamm DH5α (GIBCO/BRL) eingesetzt. Zur Isolation größerer Mengen Plasmid-DNA wurden Qiagen-tips (Qiagen) verwendet. Die Extraktion von DNA-Fragmenten aus Agarosegelen wurde mit Hilfe von Jetsorb nach Angaben des Herstellers (Genomed) durchgeführt.

Primer für PCR- und Sequenzierungs-Reaktionen wurden von der Firma Metabion bezogen. Alle Vektorkonstruktionen wurden durch AmpliTaq DNA Polymerase, FS Cycle-DNA-Sequenzierung mit Fluoreszenz-markierten Terminatoren auf einem 'ABI 373 A Sequencer' (Applied Biosystems) bestätigt.

### Transformation von Saccharomyces cerevisiae

Hefezellen, z.B. der Stamm JC34.4D (MATα, ura3-52, suc2) wurden in 10 ml YEPD (2 % Glucose; 2 % Pepton; 1 % Difco Hefeextrakt) angezogen und bei einer O.D. 600nm von 0,6 bis 0,8 geerntet. Die Zellen wurden mit 5 ml Lösung A (1M Sorbitol; 10 mM Bicin pH 8,35; 3 % Ethylenglycol) gewaschen, in 0,2 ml Lösung A resuspendiert und bei -70°C gelagert.

Plasmid DNA (5 µg) und Carrier DNA (50 µg DNA aus Herringssperma) wurden zu den gefrorenen Zellen gegeben. Die Zellen wurden anschließend durch Schütteln für 5 min bei 37°C aufgetaut. Nach Zugabe von 1,5 ml Lösung B (40 % PEG 1000; 200 mM Bicin pH 8,35) wurden die Zellen für 60 min bei 30°C inkubiert, mit 1,5 ml Lösung C (0,15 M NaCl; 10 mM Bicin pH 8,35) gewaschen und in 100 µl Lösung C resuspendiert. Die Ausplattierung erfolgte auf einem Selektivmedium mit 2 % Agar. Transformanden wurden nach einer Inkubation von 3 Tagen bei 30°C erhalten.

### Fermentation der Hefezellen

### Nährmedien

| | | |
|---|---|---|
| Vorkulturmedium: | | |
| | Bacto Yeast Nitrogen Base | 6,7 g/l |
| | Glukose x H₂O | 20 g/l |
| | KH₂PO₄ | 6,7 g/l |
| | | |
| Hauptkulturmedium: | | |
| | Glukose x H₂O | 2,0 g/l |
| | Sojapepton | 25,0 g/l |
| | (NH₄)₂SO₄ | 3,8 g/l |
| | KH₂PO₄ | 1,4 g/l |
| | MgSO₄ x 7H₂O | 1,0 g/l |
| | Thiaminhydrochlorid | 5,1 mg/l |
| | Inosit | 20 mg/l |
| | Spurenelementlösung | 3 ml/l |
| | Vitaminlösung | 3 ml/l |
| | | |
| Fütterlösung: | | |
| | (NH₄)₂SO₄ | 15,0 g/l |
| | Glukose x H₂O | 1600 g/l |
| | KH₂PO₄ | 8,7 g/l |
| | MgSO₄ x 7H₂O | 11,4 g/l |
| | Thiaminhydrochlorid | 39 mg/l |
| | Inosit | 210 mg/l |
| | Spurenelementlösung | 20,4 ml/l |
| | Vitaminlösung | 20,4 ml/l |
| | | |
| Spurenelementlösung: | | |
| | FeCl₃ x 6 H₂O | 13,5 g/l |
| | ZnCl₂ x 4 H₂O | 2,0 g/l |
| | H₃BO₃ | 0,5 g/l |
| | CoCl₂ x 6 H₂O | 2,0 g/l |
| | CuSO₄ x 5 H₂O | 1,9 g/l |
| | Na₂MoO₄ x 2 H₂O | 2,0 g/l |
| | CaCl₂ x 2 H₂O | 1,0 g/l |
| | HCl konz. | 100 ml/l |
| | | |
| Vitaminlösung: | | |
| | Riboflavin | 0,42 g/l |
| | Pantothensäure (Ca-Salz) | 5,9 g/l |
| | Nicotinsäure | 6,1 g/l |
| | Pyridoxinhydrochlorid | 1,7 g/l |
| | Biotin | 0,06 g/l |
| | Folsäure | 0,04 g/l |

### Vorkulturfermentation

Als Vorkultur wurden 200 ml Vorkulturmedium in einem 11 Erlenmeyerkolben mit 1 ml Zellsuspension aus einer Arbeitskonserve beimpft. Die Arbeitskonserven wurden in Flüssigstickstoff aufbewahrt und unmittelbar vor dem Beimpfen aufgetaut. Die Vorkultur wurde für 72 Stunden auf einem Schüttler (260 Upm) bei 28°C inkubiert.

### Hauptkulturfermentation

Als Hauptkultur wurden fed-batch Fermentationen in 101-Bioreaktoren durchgeführt. 7 Liter Hauptkulturmedium wurden mit der Vorkultur beimpft. Die Fermentationsdauer betrug 96 Stunden.

### Fermentationsbedingungen:

Temperatur: 28°C
Rührerdrehzahl: 500 Upm
Belüftung 101/min
pH: 5,5 geregelt durch Titration mit 5 N NH₄OH
Kopfraumdruck: 200 mbar

Nach 7 Stunden Fermentationszeit wurde die Fütterung gestartet. Die Fütterrate wurde so geregelt, dass ein Respiratorischer Quotient (RQ) von 1,1 gehalten wurde (RQ = gebildetes CO₂ / verbrauchter Sauerstoff). Bei einem Anstieg des RQ wurde die Fütterrate verringert, bei einem Absinken erhöht. Das Wachstum der Zellen wurde durch Messung der optischen Dichte verfolgt. Bei Ende der Fermentation wurden die Zellen durch Zentrifugation abgetrennt und das Protein aus dem Überstand isoliert.

### Reinigung von rekombinanten Interleukin-8 Muteinen

### Geräte

Die Ultra- und Diafiltration zur Aufkonzentrierung und Umpufferung der Fermenterlösung wurden mit einem System der Firma Pall-Filtron durchgeführt (5 kD Membran, Typ Omega, Filterfläche 100 cm²).

Der initiale Schritt der chromatographischen Reinigung (Anionenaustauschchromatographie an Q-Sepharose Big Beads) wurde mit einem FPLC-System der Firma Amersham-Pharmacia durchgeführt. Der verwendete Säulentyp war Amersham-Pharmacia XK 50.

Die beiden nachfolgenden Schritte der chromatographischen Reinigung (Reversed Phase Chromatographie an Vydac C8- bzw. Source 15-Material wurden an dem BioCad-System der Firma Perseptive Biosystems durchgeführt.

### Proteinchemische Charakterisierung von rekombinanten Interleukin-8 Muteinen

### Geräte

Die Sequenzanalysen wurden mit einen Proteinsequenzer Model 473A oder 494 (Procise^{™}) der Firma Applied Biosystems durchgeführt. Das Standardsequenzierungsprogramm wurde verwendet. Der Sequenzer, die verschiedenen Sequenzierungsprogramme sowie das PTH-Detektionssystem sind im Detail im Bedienungshandbuch beschrieben (User's manual protein sequencing system model 473A (1989) bzw. User's Manual Set, Protein Sequencing System Procise^{™} (1994), Applied Biosystems).

Die Reagenzien für den Betrieb des Sequenzers und die HPLC-Säule für die PTH-Detektion wurden von Applied Biosystems bezogen.

Die HPLC-Analysen wurden mit einem HP1090 HPLC-System oder HP 1100 von Hewlett Packard durchgeführt. RP-18-HPLC-Säulen (250mm x 4,6mm, 5µ-Material, 300Angström Porendurchmesser) von Backerbond oder Macherey & Nagel wurden für die Trennungen verwendet.

Die Kapillarelektrophorese Modell 270A-HT war von Applied Biosystems. Die Proben wurden in der Regel hydrodynamisch über verschiedene Zeitintervalle injiziert. Die verwendete Kapillarsäule (50µm x 72cm) war von Applied Biosystems.

Die Aminosäureanalysen wurden mit einem Aminosäureanalysator LC3000 von Eppendorf Biotronik durchgeführt. Ein leicht modifiziertes Standardtrennprogramm von Eppendorf / Biotronik wurde benutzt. Die Trennprogramme und die Funktion des Analysators sind ausführlich im Handbuch des Gerätes beschrieben.

Die Molekulargewichte wurden mit einem MALDI I System von Kratos/Shimadzu bestimmt. Die SDS-Elektrophoresen wurden mit einem Elektrophoresesystem von Pharmacia durchgeführt.

Die Gelpermeationschromatographie wurde mit einem HPLC-System von DIONEX, Modell DX-500 durchgeführt. Eine Superdex 75 GPC-Säule von Pharmacia wurde verwendet.

### Beispiele

### Beispiel 1

### Herstellung eines Hefe-Expressionsvektors zur Sekretion von Interleukin-8 TVR

Als Ausgangsmaterial für die Herstellung von Interleukin-8 TVR (Sequenz 1) diente eine synthetische humane Interleukin-8 cDNA (BBG44 von British Bio-technology Products Ltd.), die nach Insertion einer KexII-Schnittstelle über die Restriktionsenzyme HindIII und BamHI in den Vektor pA228 (Apeler et al., U.S. Pat. No. 5,707,831) kloniert wurde. In dem resultierenden Vektor pES22.9.H ist die Arg15 Aprotinin Sequenz durch die humane Interleukin-8 Sequenz (Sequenz 2) ersetzt.

Der Austausch der für ELR kodierenden Sequenz im 5'-Bereich des Gens durch eine Sequenz, die für TVR kodiert, wurde mit Hilfe der PCR-Technik unter Verwendung der Primer A und B ausgehend von pES22.9.H Plasmid DNA vorgenommen.

Der Primer A hatte folgende Sequenz:
5'GGAAGCTTGGATAAAAGAAGTGCTAAA**ACTGTCAGA**TGTCAATGCATA AAG 3' (Sequenz 3). Die HindIII Erkennungssequenz ist unterstrichen und die Codone für die Aminosäuren TVR sind fett hervorgehoben.

Der Primer B hatte folgende Sequenz:
5' CTTCTGTTCGGAGATTACCG 3' (Sequenz 4). Dieser Primer bindet im Transkriptionsterminator Bereich des Vektors.
Der PCR-Ansatz enthielt ca. 200 ng pES22.9.H Plasmid DNA, 0,2 µM Primer A, 0,2 µM Primer B, 200 µM dNTPs, 1 x PCR Reaktionspuffer (Stratagene, Opti-Prime^{™}) und 2,5 U Taq DNA Polymerase (Perkin Elmer) in einem Gesamtvolumen von 50 µl. Die 'Cycle'-Bedingungen waren: 3 min bei 94°C, 30 Zyklen mit jeweils 1,5 min bei 94°C, 1,5 min bei 55°C und 1,5 min bei 72°C und eine anschließende 5 min Inkubation bei 72°C. Der PCR-Ansatz wurde 1:4 verdünnt und mit dem Vektor pCR® 2.1-TOPO (Invitrogen) ligiert. Mit dem Ligationsansatz wurden E. coli TOP10 Zellen (Invitrogen) transformiert. Positive Klone wurden nach einem Restriktionsverdau mit den Enzymen HindIII und BamHI identifiziert und mehrere Klone sequenziert. Der Klon pIU13.10.P enthielt die gewünschte Sequenz (Sequenz 5) und wurde für die weiteren Arbeiten eingesetzt.

Der E. coli/Hefe Schaukelvektor pA202 wurde für die Konstruktion eines Hefe-Sekretionsvektors verwendet, in dem die Interleukin-8 TVR Sequenz mit der Hefe Alpha-Faktor pre-pro-Sequenz verknüpft ist. Der Vektor pA202 trägt ein Ampicillin Resistenzgen (bla) und ein URA3 Gen als selektierbare Markergene für E. coli und Hefe. Weitere essentielle Elemente des Vektors sind der Col E1 und der 2µ Ursprungspunkt der Replikation (ori). Der REP3 Locus befindet sich ebenfalls in dieser Region. Ein 1200 Bp großes EcoRI-HindIII-Fragment trägt den MFα1 Promotor und die N-terminale pre-pro-Sequenz des Hefe Alpha-Faktor-Vorläuferproteins (Kurjan und Herskowitz, Cell 30, 933 - 943, 1982). Ein BamHI-SalI Fragment des Hefe URA3 Gens fungiert als Terminationssignal für die Transkription (Yarger et al., Mol. Cell. Biol. 6, 1095 - 1101, 1986).

Durch Einführung einer modifizierten Interleukin-8 TVR cDNA als HindIII-BamHI Fragment wird die Erkennungsstelle für die KexII Protease ('Lys-Arg') innerhalb der Alpha-Faktor-pre-pro-Sequenz wieder hergestellt (WO 98/56916).

Ein ca. 240 Bp großes DNA Fragment wurde mit HindIII und BamHI aus dem Vektor pIU13.10.P herausgeschnitten, über Agarosegel-Elektrophorese gereinigt und in den ebenfalls mit HindIII und BamHI geschnittenen und dephosphorylierten Vektor pA202 kloniert. Durch diese Klonierung wird das DesPro2-Arg15-Aprotinin im Vektor pA202 durch Interleukin-8 TVR ersetzt. Hefezellen (JC34.4D) wurden mit dem aus dieser Klonierung resultierenden Vektor pIU11.11.P transformiert.

Andere E. coli/Hefe Schaukelvektoren mit unterschiedlichen Promotoren, wie z.B. der konstitutive GAPDH oder der induzierbare GAL10 Promotor, können in ähnlicher Weise hergestellt werden und führen ebenfalls zur Sekretion von Interleukin-8 TVR.

Daneben ist es selbstverständlich auch möglich, Schaukelvektoren mit anderen Hefe-Replikationsursprüngen, wie z.B. das chromosomal autonom replizierende Segment (ars), einzusetzen.

Geeignete selektierbare Markergene sind neben dem URA3 Gen solche Gene, die einer auxotrophen Hefemutante zur Prototrophie verhelfen, wie z.B. die LEU2, HIS3 oder TRP1 Gene. Außerdem können selbstverständlich auch Gene eingesetzt werden, deren Produkte Resistenz gegenüber verschiedenen Antibiotika, wie z.B. dem Aminoglykosid G418, vermitteln.
Andere Hefen, wie z.B. die methylotrophen Hefen Pichia pastoris oder Hansenula polymorpha, sind nach Transformation mit geeigneten Vektoren ebenfalls in der Lage, Interleukin-8 TVR zu produzieren.

### Beispiel 2

### Herstellung eines Hefe-Expressionsvektors zur Sekretion von Interleukin-8 DLQ

Als Ausgangsmaterial für die Herstellung von Interleukin-8 DLQ (Sequenz 6) diente eine synthetische humane Interleukin-8 cDNA (BBG44 von British Bio-technology Products Ltd.), die nach Insertion einer KexII-Schnittstelle über die Restriktionsenzyme HindIII und BamHI in den Vektor pA228 (Apeler et al., U.S. Pat. No. 5,707,831) kloniert wurde. In dem resultierenden Vektor pES22.9.H ist die Arg15 Aprotinin Sequenz durch die humane Interleukin-8 Sequenz ersetzt.

Der Austausch der für ELR kodierenden Sequenz im 5'-Bereich des Gens durch eine Sequenz, die für DLQ kodiert, wurde mit Hilfe der PCR-Technik unter Verwendung der Primer C und B ausgehend von pES22.9.H Plasmid DNA vorgenommen. Der Primer C hatte folgende Sequenz:
5'GGAAGCTTGGATAAAAGAAGTGCTAAA**GATTTGCAAT**GTCAATGCATA AAG 3' (Sequenz 7).

Die HindIII Erkennungssequenz ist unterstrichen und die Codone für die Aminosäuren DLQ sind fett hervorgehoben. Der Primer B hatte folgende Sequenz:
5' CTTCTGTTCGGAGATTACCG 3' (Sequenz 4). Dieser Primer bindet im Transkriptionsterminator Bereich des Vektors.

Der PCR-Ansatz enthielt ca. 200 ng pES22.9.H Plasmid DNA, 0,2 µM Primer C, 0,2 µM Primer B, 200 µM dNTPs, 1 x PCR Reaktionspuffer (Stratagene, Opti-Prime^{™}) und 2,5 U Taq DNA Polymerase (Perkin Elmer) in einem Gesamtvolumen von 50 µl. Die 'Cycle'-Bedingungen waren: 3 min bei 94°C, 30 Zyklen mit jeweils 1,5 min bei 94°C, 1,5 min bei 55°C und 1,5 min bei 72°C und eine anschließende 5 min Inkubation bei 72°C. Der PCR-Ansatz wurde 1:4 verdünnt und mit dem Vektor pCR® 2.1-TOPO (Invitrogen) ligiert. Mit dem Ligationsansatz wurden E. coli TOP10 Zellen (Invitrogen) transformiert. Positive Klone wurden nach einem Restriktionsverdau mit den Enzymen HindIII und BamHI identifiziert und mehrere Klone sequenziert. Der Klon pIU14.10.P enthielt die gewünschte Sequenz (Sequenz 8) und wurde für die weiteren Arbeiten eingesetzt.

Der E. coli/Hefe Schaukelvektor pA202 wurde für die Konstruktion eines Hefe-Sekretionsvektors verwendet, in dem die Interleukin-8 DLQ Sequenz mit der Hefe Alpha-Faktor pre-pro-Sequenz verknüpft ist.

Der Vektor pA202 trägt ein Ampicillin Resistenzgen (bla) und ein URA3 Gen als selektierbare Markergene für E. coli und Hefe. Weitere essentielle Elemente des Vektors sind der Col E1 und der 2µ Ursprungspunkt der Replikation (ori). Der REP3 Locus befindet sich ebenfalls in dieser Region. Ein 1200 Bp großes EcoRI-HindIII-Fragment trägt den MFα1 Promotor und die N-terminale pre-pro-Sequenz des Hefe Alpha-Faktor-Vorläuferproteins (Kurjan und Herskowitz, Cell 30, 933 - 943, 1982). Ein BamHI-SalI Fragment des Hefe URA3 Gens fungiert als Terminationssignal für die Transkription (Yarger et al., Mol. Cell. Biol. 6,1095 - 1101, 1986).

Durch Einführung einer modifizierten Interleukin-8 DLQ cDNA als HindIII-BamHI Fragment wird die Erkennungsstelle für die KexII Protease ('Lys-Arg') innerhalb der Alpha-Faktor-pre-pro-Sequenz wieder hergestellt (WO 98/56916).

Ein ca. 240 Bp großes DNA Fragment wurde mit HindIII und BamHI aus dem Vektor pIU14.10.P herausgeschnitten, über Agarosegel-Elektrophorese gereinigt und in den ebenfalls mit HindIII und BamHI geschnittenen und dephosphorylierten Vektor pA202 kloniert. Durch diese Klonierung wird das DesPro2-Arg15-Aprotinin im Vektor pA202 durch Interleukin-8 DLQ ersetzt. Hefezellen (JC34.4D) wurden mit dem aus dieser Klonierung resultierenden Vektor pIU9.11.P transformiert.

Andere E. coli/Hefe Schaukelvektoren mit unterschiedlichen Promotoren, wie z.B. der konstitutive GAPDH oder der induzierbare GAL10 Promotor, können in ähnlicher Weise hergestellt werden und führen ebenfalls zur Sekretion von Interleukin-8 DLQ. Daneben ist es selbstverständlich auch möglich, Schaukelvektoren mit anderen Hefe-Replikationsursprüngen, wie z.B. das chromosomal autonom replizierende Segment (ars), einzusetzen.

Geeignete selektierbare Markergene sind neben dem URA3 Gen solche Gene, die einer auxotrophen Hefemutante zur Prototrophie verhelfen, wie z.B. die LEU2, HIS3 oder TRP1 Gene. Außerdem können selbstverständlich auch Gene eingesetzt werden, deren Produkte Resistenz gegenüber verschiedenen Antibiotika, wie z.B. dem Aminoglykosid G418, vermitteln.

Andere Hefen, wie z.B. die methylotrophen Hefen Pichia pastoris oder Hansenula polymorpha, sind nach Transformation mit geeigneten Vektoren ebenfalls in der Lage, Interleukin-8 DLQ zu produzieren.

### Beispiel 3

### Herstellung eines Hefe-Expressionsvektors zur Sekretion von Interleukin-8 Y13H

Als Ausgangsmaterial für die Herstellung von Interleukin-8 Y13H (Sequenz 9) diente eine synthetische humane Interleukin-8 cDNA (BBG44 von British Bio-technology Products Ltd.), die nach Insertion einer KexII-Schnittstelle über die Restriktionsenzyme HindIII und BamHI in den Vektor pA228 (Apeler et al., U.S. Pat. No. 5,707,831) kloniert wurde. In dem resultierenden Vektor pES22.9.H ist die Arg15 Aprotinin Sequenz durch die humane Interleukin-8 Sequenz ersetzt.

Die Einführung der Y13H Mutation in die Interleukin-8 Sequenz wurde mit Hilfe der PCR-Technik unter Verwendung der Primer D und B ausgehend von pES22.9.H Plasmid DNA vorgenommen.

Der Primer D hatte folgende Sequenz:
5'GGAAGCTTGGATAAAAGAAGTGCTAAAGAACTTAGATGTCAATGCATA AAGACA**CAT**TCCAAACCTTTCCAC 3' (Sequenz 10). Die HindIII Erkennungssequenz ist unterstrichen und das Codon für den Y13H Austausch ist fett hervorgehoben.

Der Primer B hatte folgende Sequenz:
5' CTTCTGTTCGGAGATTACCG 3' (Sequenz 4). Dieser Primer bindet im Transkriptionsterminator Bereich des Vektors.

Der PCR-Ansatz enthielt ca. 200 ng pES22.9.H Plasmid DNA, 0,2 µM Primer D, 0,2 µM Primer B, 200 µM dNTPs, 1 x PCR Reaktionspuffer II (Perkin Elmer), 1 mM MgC12 und 2,5 U Taq DNA Polymerase (Perkin Elmer) in einem Gesamtvolumen von 100 1. Die 'Cycle'-Bedingungen waren: 3 min bei 94°C, 30 Zyklen mit jeweils 1 min bei 94°C, 1 min bei 55°C und 1 min bei 72°C und eine anschließende 5 min Inkubation bei 72°C. Der PCR-Ansatz wurde 1:4 verdünnt und mit dem Vektor pCR® 2.1-TOPO (Invitrogen) ligiert. Mit dem Ligationsansatz wurden E. coli TOP10 Zellen (Invitrogen) transformiert. Positive Klone wurden nach einem Restriktionsverdau mit dem Enzym EcoRI identifiziert und mehrere Klone sequenziert. Der Klon pIU29.6.P enthielt die gewünschte Sequenz (Sequenz 11) und wurde für die weiteren Arbeiten eingesetzt.

Der E. coli/Hefe Schaukelvektor pA202 wurde für die Konstruktion eines Hefe-Sekretionsvektors verwendet, in dem die Interleukin-8 Y13H Sequenz mit der Hefe Alpha-Faktor pre-pro-Sequenz verknüpft ist.

Der Vektor pA202 trägt ein Ampicillin Resistenzgen (bla) und ein URA3 Gen als selektierbare Markergene für E. coli und Hefe. Weitere essentielle Elemente des Vektors sind der Col E1 und der 2µ Ursprungspunkt der Replikation (ori). Der REP3 Locus befindet sich ebenfalls in dieser Region. Ein 1200 Bp großes EcoRI-HindIII-Fragment trägt den MFα1 Promotor und die N-terminale pre-pro-Sequenz des Hefe Alpha-Faktor-Vorläuferproteins (Kurjan und Herskowitz, Cell 30, 933 - 943, 1982). Ein BamHI-SalI Fragment des Hefe URA3 Gens fungiert als Terminationssignal für die Transkription (Yarger et al., Mol. Cell. Biol. 6, 1095 - 1101, 1986).

Durch Einführung einer modifizierten Interleukin-8 Y13H cDNA als HindIII-BamHI Fragment wird die Erkennungsstelle für die KexII Protease ('Lys-Arg') innerhalb der Alpha-Faktor-pre-pro-Sequenz wieder hergestellt (WO 98/56916).

Ein ca. 240 Bp großes DNA Fragment wurde mit HindIII und BamHI aus dem Vektor pIU29.6.P herausgeschnitten, über Agarosegel-Elektrophorese gereinigt und in den ebenfalls mit HindIII und BamHI geschnittenen und dephosphorylierten Vektor pA202 kloniert. Durch diese Klonierung wird das DesPro2-Arg15-Aprotinin im Vektor pA202 durch Interleukin-8 Y13H ersetzt. Hefezellen (JC34.4D) wurden mit dem aus dieser Klonierung resultierenden Vektor pIU16.7.P transformiert.

Andere E. coli/Hefe Schaukelvektoren mit unterschiedlichen Promotoren, wie z.B. der konstitutive GAPDH oder der induzierbare GAL10 Promotor, können in ähnlicher Weise hergestellt werden und führen ebenfalls zur Sekretion von Interleukin-8 Y13H.

Daneben ist es selbstverständlich auch möglich, Schaukelvektoren mit anderen Hefe-Replikationsursprüngen, wie z.B. das chromosomal autonom replizierende Segment (ars), einzusetzen.

Geeignete selektierbare Markergene sind neben dem URA3 Gen solche Gene, die einer auxotrophen Hefemutante zur Prototrophie verhelfen, wie z.B. die LEU2, HIS3 oder TRP1 Gene. Außerdem können selbstverständlich auch Gene eingesetzt werden, deren Produkte Resistenz gegenüber verschiedenen Antibiotika, wie z.B. dem Aminoglykosid G418, vermitteln.

Andere Hefen, wie z.B. die methylotrophen Hefen Pichia pastoris oder Hansenula polymorpha, sind nach Transformation mit geeigneten Vektoren ebenfalls in der Lage, Interleukin-8 Y13H zu produzieren.

### Beispiel 4

### Reinigung von rekombinanten humanen Interleukin-8 Muteinen

10 1 Fermentationsüberstand wurden durch Ultrafiltration über eine 5 kD Membran (Typ Omega, Fa. Pall-Filtron) aufkonzentriert (1/7) und anschließend durch Diafiltration in 20 mM Ethanolamin, pH 9.5 (Puffer A) umgepuffert. Der Ansatz wurde auf eine Chromatographiesäule, gefüllt mit 40 ml Q-Sepharose Big Beads (Fa. Amersham-Pharmacia) aufgetragen, die zuvor mit Puffer A äquilibriert wurde. Die Säule wurde mit Puffer A nachgewaschen und anschließend mit Puffer B (Puffer A + 1 M NaCl) im Stufengradienten eluiert. Im nächsten Schritt wurde der Durchlauf, in dem Interleukin-8 nachgewiesen wurde, mit 0,1 % TFA versetzt und auf eine 100 ml Vydac C8-Säule (Muder + Wochele) gepumpt, die zuvor mit 0,1 % TFA in Wasser (Puffer C) äquilibriert wurde. Nach dem Nachwaschen mit Puffer C erfolgte die lineare Gradienten-Elution mit 80 % ACN + 0,1 % TFA (Puffer D). (Gradient 0 % B bis 100 % B in 6 Säulenvolumina, Flussrate 20 ml/min, IL-8 und Muteine eluieren bei etwa 44 % B). Fraktionen, in denen IL-8 nachgewiesen wurde, wurden vereinigt, mit Wasser auf 20 % ACN verdünnt und lyophilisiert.. Nach dem Rekonstituieren des Lyophilisates in Puffer C wurde die Lösung auf eine 100 ml Source 15 Reversed Phase Säule (Fa. Amersham-Pharmacia) aufgetragen, die zuvor mit Puffer C äquilibriert wurde. Nach dem Nachwaschen mit Puffer C erfolgte die lineare Gradienten-Elution mit Puffer D. (Gradient 0 % B bis 100 % B in 9 Säulenvolumina, Flussrate 27 ml/min, IL-8 und Muteine eluieren bei etwa 44 % B). Fraktionen, in denen IL-8 nachgewiesen wurde, wurden vereinigt, mit Wasser auf 20 % ACN verdünnt und lyophilisiert.

### Beispiel 5

### N-terminale Sequenzanalyse

0,5nmol in Wasser gelöste Interleukin-8 Muteine wurden auf ein Sequenzer-sheet, das mit Polybren^{R} vorinkubiert war, geladen. Die Proteine wurden mit dem normalen Sequenzerzyklus sequenziert. Die PTH-Aminosäuren wurden mittels Online-HPLC mit Hilfe eines 50pmol PTH-Standards identifiziert.

Die Interleukin-8 Muteine wurden über 4 - 20 Stufen sequenziert. Die Abb.1 zeigt die bestimmten Proteinsequenzen. Sie sind identisch mit den klonierten Sequenzen.

**Abb. 1: Sequenzanalyse von Interleukin-8-Muteinen.**

| Interleukin-8-Mutein | Bestimmte N-terminale Sequenz |
|---|---|
| TVR-Mutein | Ser-Ala-Lys-**Thr-Val-Arg**-Cys-Gln-Cys-Ile-Lys-Thr-Tyr-Ser-Lys-Pro-Phe-His-Pro-Lys... |
| DLQ-Mutein | Ser-Ala-Lys-**Asp-Leu-Gln**-Cys-Gln-Cys-Ile-Lys-Thr-Tyr-Ser-Lys-Pro-Phe-His-Pro-Lys... |
| Y13H-Mutein | Ser-Ala-Lys-Glu-Leu-Arg-Cys-Gln-Cys-Ile-Lys-Thr-**His**-Ser-Lys... |

### Beispiel 6

### Aminosäureanalyse

Die Aminosäureanalyse stellt einen wichtigen qualitativen und quantitativen Parameter für die Charakterisierung von Proteinen dar. Neben dem Proteingehalt wird bei bekannter Primärstruktur die Anzahl der Einzelaminosäuren ermittelt. Die Aminosäureanalyse von Interleukin-8 Muteinen ist in guter Übereinstimmung mit den theoretischen Werten aus der Primärstruktur (Tab.1).

100µg der Interleukin-8 Muteine wurden in 200µl 6N HCl gelöst und 1h bei 166°C hydrolysiert. Ca. 1nmol der Proben wurde auf den Aminosäureanalysator gegeben. Die Menge an Aminosäure wurde über einen 4nmol Aminosäurestandard ermittelt.

**Tabelle 1: Aminosäureanalyse von TVR, DLQ und Y13H IL-8 Mutein. Die ganzen Zahlen sind auf Phe=3, Ala=3oder Asx=5 bezogen.**

| Aminosäure | TVR IL-8 | theoret. Wert | DLQ IL-8 | theoret. Wert | Y13H IL-8 | theoret. Wert |
|---|---|---|---|---|---|---|
| CysSO₃H* | 4,00 | 4 | 4,00 | 4 | 4,01 | 4 |
| Asx | 5,18 | 5 | 5,70 | 6 | 5,00 | 5 |
| Thr | 2,85 | 3 | 1,95 | 2 | 2,22 | 2 |
| Ser | 4,07 | 5 | 3,75 | 5 | 4,21 | 5 |
| Glx | 8,71 | 9 | 9,37 | 10 | 9,76 | 10 |
| Gly | 2,16 | 2 | 2,01 | 2 | 2,19 | 2 |
| Ala | 3,00 | 3 | 3,10 | 3 | 3,12 | 3 |
| Val | 5,50 | 6 | 4,70 | 5 | 4,71 | 5 |
| Met | - | - | - | - | - | - |
| Ile | 4,32 | 5 | 4,15 | 5 | 4,33 | 5 |
| Leu | 5,18 | 5 | 5,83 | 6 | 6,02 | 6 |
| Tyr | 1,32 | 1 | 1,41 | 1 | - | - |
| Phe | 3,01 | 3 | 3,00 | 3 | 2,98 | 3 |
| His | 1,84 | 2 | 1,77 | 2 | 2,75 | 3 |
| Lys | 8,58 | 9 | 8,63 | 9 | 8,84 9 | |
| Arg | 4,64 | 5 | 3,79 | 4 | 4,69 | 5 |
| Pro | 4,17 | 4 | 4,18 | 4 | 4,12 | 4 |
| Trp | n.d. | 1 | n.d. | 1 | n.d. | 1 |

| | | | | | | |
|---|---|---|---|---|---|---|
| *Cysteine wurde nach der Perameisensäureoxidation bestimmt. | | | | | | |

### Beispiel 7

### Molekulargewichtsbestimmung

Ca. 1µg der Interleukin-8 Muteine wurde mit der MALDI-Technik analysiert. Als Matrix wurde Sinapinsäure verwendet. Die Standardproteine für die Massenkalibrierung waren Rinderinsulin, Cytochrom C und Melittin.

Das ermittelte Molekulargewicht ist dabei in guter Übereinstimmung mit dem theoretischen Wert der jeweiligen Muteine im Rahmen der Genauigkeit der Messmethode. Die Tabelle 2 zeigt die bestimmten Molekulargewichte im Vergleich mit den theoretischen Werten.

**Tabelle 2: Molekulargewichte der gereinigten IL-8 Muteine und des IL-8 Wildtyps**

| **Interleukin-8 Mutein** | **Molekulargewicht in Da** | **Theoret. Molekulargewicht in Da** |
|---|---|---|
| TVR IL-8 | 8342±4 | 8343 |
| DLQ IL-8 | 8342±3 | 8343 |
| Y13H IL-8 | 8355±4 | 8359 |
| IL-8 Wildtyp (Met-Form) | 8521±4 | 8517 |

### Beispiel 8

### SDS-Gelelektrophorese

Die Interleukin-8 Muteine TVR, DLQ, Y13H und die Met-Form des IL-8 Wildtyps wurden mittels SDS-Elektrophorese unter reduzierenden und nicht reduzierenden Bedingungen analysiert. Die SDS-Gelelektrophorese wurde nach den Bedingungen von Laemmli (Laemmli, Nature 227, 680 - 685 1970) durchgeführt. Die Interleukin-8 Muteine wurden in einem 10-20%-igen SDS-Gel aufgetrennt und mittels Coomassie Brilliant Blue R-250 bzw. der Silberfärbung (Merril et.al., Anal. Biochem. 110, 210 - 207 1981) angefärbt. Im Falle der Coomassie Blue Färbung wurden ca. 30µg und bei der Silberfärbung ca. 5µg eingesetzt.

In den SDS-Gelen laufen die IL-8 Muteine und der Wildtyp als einzelne Banden bei ca.9 kD.

### Beispiel 9

### Kapillarelektrophorese

Die Kapillarelektrophorese erlaubt die Trennung von Peptiden und Proteinen aufgrund ihrer Ladung im elektrischen Feld. Die Güte der Trennung hängt dabei vom Puffer, dem pH-Wert, der Temperatur und den verwendeten Additiven ab. Als Kapillaren werden sogenannte "fused silica" Säulen mit einem Innendurchmesser von 50-100µm eingesetzt. Die Interleukin-8 Muteine wurden an einer "fused silica" Säule im elektrischen Feld getrennt. Ca. 8ng der Interleukin-8 Muteine wurden an einer Glassäule (Länge 72cm, Innendurchmesser 50µm) untersucht. Bedingungen: Stromstärke 90µA, Säulentemperatur 25°C, 100mM Phosphatpuffer pH 3.0, Detektion 210nm, Aufgabe unter Druck 3 Sek.

Im Elektropherogramm eluieren die Interleukin-8 Muteine als ein Hauptpeak mit einer Retentionszeit von ca. 10min.

### Beispiel 10

### Reverse Phase Chromatographie

Bei der HPLC-Chromatographie von Proteinen an chemisch gebundene Umkehrphasen kommt es über eine hydrophobe Wechselwirkung der Proteine zu einer Bindung an die verwendete Phase. Die Proteine werden entsprechend der Stärke ihrer Bindung an die stationäre Phase von organischen Lösungsmitteln (mobile Phase) verdrängt.

Aus diesem Grund ist diese Methode ein gutes Kriterium für die Beurteilung der Reinheit eines Proteins.

Ca. 3 nmol der Interleukin-8 Muteine wurden an einer Nucleosil RP-18-HPLC-Säule (5µ-Material, 4,6mmx250mm, 300Angström Porengröße) chromatographiert. Als Eluent wurde ein Acetonitril / TFA-Gradient verwendet. Bedingungen: Fluss 0,7ml/min, Säulentemperatur 40°C, Detektion 210nm, Lösungsmittel A 0,1%TFA, Lösungsmittel B 0,1%TFA / 60% Acetonitril; Gradient: 0min. 0%B, 10min. 0%B, 70min. 100%B, 80min. 0%B.

Die Interleukin-8-Muteine eluieren als Peak von der RP-18-Phase mit Retentionszeiten von ca. 52min (TVR IL-8), ca. 53min (DLQ IL-8), ca. 52min (Met-Wildtyp IL-8), ca. 52min (Y13H IL-8).

### Beispiel 11

### Gelpermeationschromatographie

Ca. 50µg der Interleukin-8 Muteine TVR, DLQ und Y13H wurden mittels Gelpermeationschromatographie an einer Superdex 75 Säule chromatographiert. Als Puffer wurde 600mM NaCl/50mM Na₂HPO₄ pH 7,0 verwendet; Fluss 0,5ml/min.; Detektion 215nm; Raumtemperatur. Als Standardproteine für die Molekulargewichtskalibrierung der Säule wurden Rinderserumalbumin, Ovalbumin, Carbonic Anhydrase, Myoglobin, Cytochrom C, Aprotinin und Tyrosin eingesetzt.

Die Interleukin-8 Muteine eluieren mit einer Retentionszeit von ca. 25 Minuten. Dies entspricht einem relativen Molekulargewicht (nach der Kalibrierung der Säule mit globulären Proteinen) von ca. 17kD in Lösung. Die IL-8 Muteine liegen in der verwendeten Lösung als Dimere vor.

## Patentansprüche

1. Verwendung von IL-8 Y13H in Screening-Ansätzen zum Auffinden nieder- und hochmolekularer IL-8 Antagonisten für die IL-8 Rezeptoren CXCR1 und CXCR2.

2. Verwendung von im Vergleich zu Wildtyp IL-8 schwächer affinen IL-8 Muteinen in Screening-Ansätzen zum Auffinden nieder- und hochmolekularer IL-8 Antagonisten für die IL-8 Rezeptoren CXCR1 und CXCR2.

## Claims

1. Use of IL-8 Y13H in screening assays for finding low and high molecular weight IL-8 antagonists for the IL-8 receptors CXCR1 and CXCR2.

2. Use of IL-8 muteins which have weaker affinity than wild-type IL-8 in screening assays for finding low and high molecular weight IL-8 antagonists for the IL-8 receptors CXCR1 and CXCR2.

## Revendications

1. Utilisation de la mutéine Y13H de l'IL-8 dans des essais de screening pour la recherche d'antagonistes de l'IL-8 de bas et haut poids moléculaires pour les récepteurs CXCR1 et CXCR2 de l'IL-8.

2. Utilisation de mutéines de l'IL-8 de plus faible affinité comparativement à l'IL-8 de type sauvage dans des essais de screening pour la recherche d'antagonistes de l'IL-8 de bas et haut poids moléculaires pour les récepteurs CXCR1 et CXCR2 de l'IL-8.
